(19) Europäisches Patentamt
European Patent Office
Office européen des brevets

(11) **EP 3 072 500 B1**

(12) **EUROPEAN PATENT SPECIFICATION**

(45) Date of publication and mention of the grant of the patent:
**10.07.2019 Bulletin 2019/28**

(51) Int Cl.:
*A61Q 17/04* (2006.01)   *A61K 8/29* (2006.01)
*A61K 8/37* (2006.01)   *A61K 8/49* (2006.01)
*A61Q 1/02* (2006.01)   *A61K 8/02* (2006.01)
*A61K 8/06* (2006.01)   *A61K 8/81* (2006.01)

(21) Application number: **15851616.1**

(22) Date of filing: **12.08.2015**

(86) International application number:
**PCT/KR2015/008461**

(87) International publication number:
**WO 2016/114466 (21.07.2016 Gazette 2016/29)**

(54) **COSMETIC PRODUCT COMPRISING WATER-INSOLUBLE SPONGE IMPREGNATED WITH COSMETIC COMPOSITION HAVING ULTRAVIOLET RAY BLOCKING FUNCTION**

KOSMETISCHES PRODUKT MIT EINEM WASSERUNLÖSLICHEN SCHWAMM MIT IMPRÄGNIERUNG MIT EINER KOSMETISCHEN ZUSAMMENSETZUNG MIT UV-STRAHLENBLOCKIERUNGSFUNKTION

PRODUIT COSMÉTIQUE COMPRENANT UNE ÉPONGE INSOLUBLE DANS L'EAU IMPRÉGNÉE D'UNE COMPOSITION COSMÉTIQUE AYANT COMME FONCTION LE BLOCAGE DES RAYONS ULTRAVIOLETS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.01.2015 KR 20150004142**
**28.01.2015 KR 20150013647**
**04.08.2015 KR 20150109816**

(43) Date of publication of application:
**28.09.2016 Bulletin 2016/39**

(73) Proprietor: **LG Household & Health Care Ltd.**
**Seoul 03184 (KR)**

(72) Inventors:
• **PARK, Byeong-Gyu**
**Daejeon 34114 (KR)**
• **KANG, Sung-Soo**
**Daejeon 34114 (KR)**
• **PARK, Sang-Wook**
**Daejeon 34114 (KR)**
• **KIM, Kyong-Seob**
**Daejeon 34114 (KR)**

(74) Representative: **Zacco GmbH**
**Bayerstrasse 83**
**80335 München (DE)**

(56) References cited:
**EP-A1- 1 616 553**   **JP-A- 2002 284 638**
**JP-A- 2006 241 150**   **KR-A- 20130 116 205**
**KR-A- 20130 116 205**   **KR-A- 20140 004 575**
**KR-A- 20140 143 730**   **US-A1- 2004 170 670**
**US-A1- 2014 023 689**

• **DATABASE GNPD [Online] MINTEL; 1 February 2013 (2013-02-01), Procter & Gamble: "Stay Fabulous 3in1 Foundation + Sunscreen SPF 20", XP002772473, Database accession no. 1993209**
• **Anonymous: "Erfahrungsberichte Make-Up ebelin Make-up Schwämmchen oval : " Oval zum perfekten Teint :)"", , 10 September 2012 (2012-09-10), pages 1-3, XP055393265, Retrieved from the Internet: URL:http://www.ciao.de/ebelin_Make_up_Schw ammchen_oval__Test_8862890 [retrieved on 2017-07-24]**

**Description**

<u>TECHNICAL FIELD</u>

The present disclosure relates to a cosmetic composition having an excellent ultraviolet (UV) blocking function that is impregnated in a water insoluble sponge. More particularly, to address and solve a reduction problem of a UV blocking effect based on the total content of a UV blocking agent included in a cosmetic composition impregnated in a water insoluble sponge, the present disclosure relates to a cosmetic composition having an excellent UV blocking effect and cosmetics including the cosmetic composition impregnated in a water insoluble sponge.

<u>BACKGROUND ART</u>

Recently, as the ozone layer is deteriorating due to the environmental pollution, exposure to ultraviolet (UV) rays is getting intense, and UV rays act as a main cause of erythema, edema, freckles, and skin cancers on the skin. More recently, development of products that protect the skin from UV rays is being needed. Particularly, with the growing affluence in life, the desire to protect the skin from daily UV radiation is increasing, and with the increasing people doing leisure activities such as ski, golf, and open water swimming, the demand for skin protection from UV rays is increasing, and to meet the demand, many studies are being conducted.

Generally, UV rays are classified into three regions according to the wavelength; 200-290 nm wavelength as UV C (UVC), 290-320 nm wavelength as UV B (UVB), and 320-400 nm wavelength as UV A (UVA). Among them, UVC is filtered before reaching the earth's surface through the ozone layer, and UVB penetrates through the epidermis of the skin, causing erythema, freckles, and edema. It is known that UVA penetrates through the dermis of the skin, causing skin cancers, wrinkles, and acceleration of the production of melanin, inducing skin aging and skin irritation.

The long wavelength UV (UVA) plays its role in modification of protein in the dermis layer, hemotelangiosis and destruction of nucleic acid (DNA), causing accelerated skin aging, so the skin should be protected from UVA. On the other hand, most of the middle wavelength UV (UV-B) is absorbed in the epidermis part of the skin and affects the epidermis quickly, causing sunburns, so UV-B is found harmful. The skin is exposed to the long wavelength UV all the year round, but because there is no subjective symptom, the skin gradually ages in unconscious manner.

Since the danger of the exposure to sunlight was reported, people's interest in UV protection products has increased, and to keep up with the trend, products having a variety of sun protection factors (SPF) and protection grades of UVA (PA) are being released.

On the other hand, in relation to the formulation of cosmetics, liquid-type cosmetics are highly preferred due to good performance of application or feeling upon usage, but have disadvantages - inconvenience caused by spreading and rubbing it in hands to use and portability restriction. To solve the problem, cosmetics convenient to use and carry with have been developed in which a liquid-type cosmetic composition in low viscosity emulsion formulation is impregnated in a water insoluble sponge produced by foaming styrene-butadiene rubber (SBR), butadiene rubber (BR), acrylonitrile-butadiene rubber (NBR), ABS, polyethylene (PE), polyvinyl alcohol (PVA), ethylene vinyl acetate (EVA), polyurethane (PU), and among them, cosmetics impregnated in a polyurethane sponge are being widely used.

KR20130116205A (US2015118269A1) relates to a cosmetic composition carrier in which one or more foams having a number of pores in the range of 10 ppi to 130 ppi are impregnated with a cosmetic composition having a viscosity of 1,000 to 5,000 cps and 15,000 to 100,000 cps to create a compact type liquid cosmetic composition.
The product description of Procter & Gamble "Stay Fabulous 3 in 1 Foundation + Sunscreen SPF 20" of 2013 discloses a foundation including UV blocking components to be included in a pumping type bottle. It does not disclose an impregnation material.

In these circumstances, the inventors attempted to impart a UV blocking function to a cosmetic composition impregnated in a water insoluble sponge, but the inventors found that when a cosmetic composition having a UV blocking function is impregnated into a water insoluble sponge, there is a problem with an actual sun protection factor/grade that is lower than expected from the total amount of the UV blocking agent included in the cosmetic composition.

Further, the inventors identified the cause of the problem - the oil soluble organic UV blocking agent in direct contact with the water insoluble sponge is adsorbed to the water insoluble sponge, and as a result, the UV blocking agent is not delivered at the same concentration as before impregnation.

<u>DISCLOSURE</u>

<u>Technical Problem</u>

The present disclosure is directed to providing a cosmetic composition in which by selecting the composition of the ultraviolet (UV) blocking agent and/or the phase of the cosmetic composition to prevent the adsorption of an oil soluble organic UV blocking agent to a water insoluble sponge, the imparted UV blocking function is thereby fully exerted, an excellent UV blocking function (SPF 30 or above, PA++ or above) is exerted and a whitening problem is eliminated, and cosmetics including the cosmetic composition impregnated in the water insoluble sponge and a method for preparing the cosmetic composition and/or cosmetics.

More specifically, the present invention is directed at a cosmetic product comprising a water insoluble sponge and a cosmetic composition to be impregnated in said water insoluble sponge, wherein said cosmetic composition comprises one or more of the following UV blocking agents:

(i) a water soluble or water dispersible organic UV blocking agent; and
(ii) an encapsulated organic UV blocking agent,

and an oil soluble organic UV blocking agent is present in less than 10 wt% per the total weight of the organic ultraviolet light blocking agent included in the cosmetic composition, wherein the formulation of the cosmetic composition is a water-in-oil emulsion, wherein said water insoluble sponge is formed of a hydrophobic monomer polymer.

There is further disclosure directed to a cosmetic composition - in general - including a UV blocking agent, wherein the cosmetic composition is impregnated in a water insoluble sponge, and an oil soluble organic UV blocking agent is substantially absent in the external phase of the cosmetic composition or an oil soluble organic UV blocking agent is present in the external phase but not impregnated in the water insoluble sponge.

Also, there is provided the product according to the invention with a cosmetic composition having SPF 30 or above and PA++ or above.

Also, there is provided the product according to the invention with the cosmetic composition wherein the UV blocking agent is an inorganic UV blocking agent.

Also, there is provided the product according to the invention with the cosmetic composition wherein the UV blocking agent is at least one of the following:

(a) a water soluble or water dispersible organic UV blocking agent;
(b) an (not claimed) organic UV blocking agent with inversion from water insoluble to water soluble or water dispersible; and
(b) an encapsulated organic UV blocking agent.

There is also - in general and not claimed - provided a cosmetic composition of which the formulation is a single water phase, an oil-in-water type emulsion, or multiple emulsions in which the external phase is water.

Also, there is - in general and not claimed - provided a cosmetic composition of which the formulation is oil-in-water type or multiple emulsions, and the oil soluble organic UV blocking agent is present outside the external phase of the cosmetic composition.

As said above the product according to the invention with the cosmetic composition in the composition the water insoluble sponge is formed of a hydrophobic monomer polymer. This could for example be including styrene-butadiene rubber (SBR), butadiene rubber (BR), natural rubber (NR) or acrylonitrile-butadiene rubber (NBR).

Also, there is provided the product according to the invention with the cosmetic composition which is foundation.

Further, there is general disclosure of cosmetics including a water insoluble sponge and a cosmetic composition impregnated in the water insoluble sponge, wherein the cosmetic composition includes a UV blocking agent, and an oil soluble organic UV blocking agent is substantially absent in the external phase or an oil soluble organic UV blocking agent is present in the external phase but not adsorbed to the water insoluble sponge.

As said above, in the product according to the invention includes a water insoluble sponge and the cosmetic composition impregnated in the water insoluble sponge, wherein the cosmetic composition includes at least one of the following organic UV blocking agents:

(a) a water soluble or water dispersible organic UV blocking agent;
(b) an (not claimed) organic UV blocking agent with inversion from water insoluble to water soluble or water dispersible; and
(b) an encapsulated organic UV blocking agent, and

said oil soluble organic UV blocking agent being present in less than 10% per the total weight of the organic UV blocking agent included in the cosmetic composition.

Also, there are provided the product according to the invention with the cosmetic composition which further includes an inorganic UV blocking agent.

Also, there are provided a non-claimed cosmetics wherein the formulation of the cosmetic composition is a single water phase, an oil-in-water type emulsion, or multiple emulsions in which the external phase is water.

Also, there is provided the product according to the invention with the cosmetics wherein the water insoluble sponge is formed of a hydrophobic monomer polymer including styrene-butadiene rubber (SBR), butadiene rubber (BR), natural rubber or acrylonitrile rubber (NR) or acrylonitrile-butadiene rubber (NBR).

Also, there is provided the product according to the invention wherein the cosmetic composition is foundation.

Technical Solution

To achieve the above object, the present disclosure provides a cosmetic composition having a composition and/or phase for preventing the adsorption of an oil soluble organic ultraviolet (UV) blocking agent to a water insoluble sponge.

More specifically, the present disclosure provides a cosmetic product with a cosmetic composition as described above producing an excellent UV blocking effect, preferably with SPF 30 or above and PA++ or above. In addition, the present disclosure provides a method for preparing the cosmetic composition.

[Cosmetic composition]

The cosmetic composition according to the present disclosure includes a UV blocking agent.

The term "UV blocking agent" as used herein includes any component capable of blocking UV rays that reach on the skin by scattering, reflection, or absorption, for example, a (non-claimed) inorganic UV blocking agent, an organic UV blocking agent, and (non-claimed) mixtures thereof.

The cosmetic product according to the invention with the cosmetic composition will include an organic UV blocking agent.

The organic UV blocking agent has advantages - it has a UV absorption effect and is less sensitive to whitening, and the organic UV blocking agent can be divided into (non-claimed) oil soluble, water soluble, oil dispersible, and water dispersible organic UV blocking agents. For example, the (non-claimed) oil soluble or (non-claimed) oil dispersible organic UV blocking agent includes, but is not limited to, 4-methylbenzylidene camphor, disodiumphenyldibenzimidazole tetrasulfonate, diethylhexyl butamido triazone, methylene bis-benzotriazolyl tetramethylbutylphenol, ethylhexyl dimethyl paba, glyceryl paba, drometrizole, drometrizole trisiloxane, digalloyl trioleate, 3-(4-methylbenzylidene)-camphor, meth-ylantranylate, bis-ethylhexyloxyphenolmethoxyphenyltriazine, diethylaminohydroxybenzoylhexylbenzoate, benzophe-none-3, benzophenone-4, benzophenone-8, butylmethoxydibenzoylmethane, cinoxate, ethylhexyltriazone, octylmethoxycinnamate, octocrylene, octyl dimethyl paba, octyl salicylate, oxybenzone, cinoxate, octyltriazone, ethylhexylmethoxycinnamate, ethylhexylsalicylate, para aminobenzoic acid, homosalate, isoamyl-P-methoxycinnamate, bisethylhexyloxyphenolmethoxyphenyltriazine, and polysilicone-15, and the water soluble or water dispersible organic UV blocking agent includes, but is not limited to, terephthalylidene dicamphor sulfonic acid and its salts, 2-phenylbenzimidazole-5-sulfonic acid.

Also, the inorganic UV blocking agent has UV scattering and reflection effects but is sensitive to whitening. For example, the inorganic UV blocking agent includes, but is not limited to, titanium dioxide, zinc oxide, zirconium oxide, and calcium cerium oxide.

The inventors explained, in the cosmetic composition of the product according to the present invention, the reason that the imparted UV blocking function is not fully exerted is because an oil soluble organic UV blocking agent is adsorbed to the water insoluble sponge, and they attempted to solve the problem through the control of the amount or morphology of the oil soluble organic UV blocking agent in the external phase of the cosmetic composition.

Accordingly, UV blocking agents other than the organic UV blocking agent already included in the cosmetic composition of the present disclosure may be optimally selected by those skilled in the art in selecting the type/dispersibility (e.g. including water soluble or water dispersible organic UV blocking agents, organic UV blocking agents with inversion from water insoluble to water soluble or water dispersible, or inorganic UV blocking agents), the amount or position in the cosmetic composition irrespective of adsorption to the water insoluble sponge.

The following other UV blocking agents are existing: any UV blocking agent generally used in the art irrespective of the type/dispersibility/water·oil soluble of the UV blocking agent (e.g. including oil soluble or oil dispersible organic UV blocking agents, water soluble or water dispersible organic UV blocking agents, organic UV blocking agents with inversion from water insoluble to water soluble or water dispersible or inorganic UV blocking agent, that is, even including oil soluble or oil dispersible organic UV blocking agents) and the amount of the UV blocking agent.

The term "impregnation" as used herein refers to manipulation for permeation, and can be interchangeably used with the terms penetration, adhesion, and absorption commonly used in the art, and more specifically, refers to a state in which the water insoluble sponge contains the cosmetic composition. However, the degree of release, desorption, separation or discharge of the impregnated material (the cosmetic composition) (from the water insoluble sponge) is limited by the physical pressure (e.g., 3 bar or less) applied to apply the cosmetic composition to the skin. The physical pressure generally applied to apply the cosmetic composition to the skin may be applied using a hand or tool (e.g. a puff).

The term "adsorption" as used herein refers to adhesion, combination, binding, or concentration of a material, and more specifically, refers to adhesion, combination, binding, or concentration of an (e.g. oil soluble) organic UV blocking agent to the water insoluble sponge, and accordingly, refers to that the adsorbed state (of the oil soluble organic UV blocking agent to the water insoluble sponge) is maintained by the physical pressure (e.g., 3 bar or less) generally applied to apply the cosmetic composition to the skin and the material adsorbed by the physical pressure (the oil soluble organic UV blocking agent) is not released, desorbed, separated or discharged (from the water insoluble sponge). The physical pressure generally applied to apply the cosmetic composition to the skin may be applied using a hand or tool (e.g. a puff).

As a general description, an oil soluble organic UV blocking agent may be substantially absent in the external phase of a cosmetic composition.

The term "external phase" as used herein refers to a part having the largest direct contact surface with the water insoluble sponge when the cosmetic composition is impregnated in the water insoluble sponge. For example, when an emulsion consists of a single phase (i.e., a single water phase or a single oil phase), the corresponding single phase serves as the external phase, and in a water-in-oil type emulsion, the external phase is oil, and in an oil-in-water type emulsion, the external phase is water, and when multiple emulsions are defined as '(X)/(X)n/Y' (the 'X, Y' may be water phase or oil phase, and the water phase is shortened to 'W' and the oil phase is shortened to 'O'. 'n' is larger than or equal to 1), the 'Y' corresponds to the external phase, and for example, the external phase of the oil-in-water-in-oil ($O_1$/W/$O_2$) type is 'oil ($O_2$)'.

The term "substantially absent" as used herein refers to that the amount present in the external phase of a cosmetic composition is less than 10 wt%, less than 9 wt%, less than 8 wt%, less than 7 wt%, less than 6 wt%, less than 5 wt%, less than 4 wt%, less than 3 wt%, less than 2 wt%, less than 1 wt%, less than 0.5 wt%, less than 0.05 wt%, less than 0.0005 wt%, or most preferably less than 0.00001 wt% per the total weight included in the cosmetic composition. For example, the amount of the oil soluble organic UV blocking agent included in the external phase of a generally described cosmetic composition may be less than 10 wt%, less than 9 wt%, less than 8 wt%, less than 7 wt%, less than 6 wt%, less than 5 wt%, less than 4 wt%, less than 3 wt%, less than 2 wt%, less than 1 wt%, less than 0.5 wt%, less than 0.05 wt%, less than 0.0005 wt%, or most preferably less than 0.00001 wt% per the total amount of the organic UV blocking agent included in the cosmetic composition. In general, the term may cover the case where only an inorganic UV blocking agent is present as the UV blocking agent in the external phase and an organic UV blocking agent is absent. In another general disclosure, the term may cover the case where only a water soluble, water dispersible organic UV blocking agent is present as the UV blocking agent in the external phase and an oil soluble organic UV blocking agent is absent.

In another embodiment, an oil soluble organic UV blocking agent non-adsorbed to the water insoluble sponge may be present in the external phase of a cosmetic composition.

The term "non-adsorbed" as used herein refers to that a value calculated by 'the weight of the (oil soluble organic) UV blocking agent adsorbed to the water insoluble sponge'/'the weight of the (oil soluble organic) UV blocking agent included in the external phase of the cosmetic composition' x 100 (%) is less than 10 wt%, less than 9 wt%, less than 8 wt%, less than 7 wt%, less than 6 wt%, less than 5 wt%, less than 4 wt%, less than 3 wt%, less than 2 wt%, less than 1 wt%, less than 0.5 wt%, less than 0.05 wt%, less than 0.0005 wt%, or most preferably less than 0.00001 wt%, and the (oil soluble organic) UV blocking agent refers to at least one (oil soluble organic) UV blocking agent included in a cosmetic composition.

The general expression: "oil soluble organic UV blocking agent present in the external phase and non-adsorbed to the water insoluble sponge" may be embodied by the phase control of a cosmetic composition and/or selection of a composition of an oil soluble organic UV blocking agent.

In selecting the phase of a cosmetic composition, when the external phase of the cosmetic composition consists of water phase, the effect as disclosed above can be achieved. The formulation of a cosmetic composition in which the external phase is water may include, for example, single water phase (water phase alone), oil-in-water type, or multiple emulsions ('(X)/(X)n/Y') in which Y is water phase (i.e., multiple emulsions in which the external phase is water) as described above. When the external phase of a cosmetic composition is water, the oil soluble organic UV blocking agent present in the water external phase is preferably water soluble or water dispersible ones or those with inversion to water soluble or water dispersible, and if it is oil soluble or oil dispersible, those modified to be dispersible in water by a method such as encapsulation are desirable.

Also, in selecting the composition of the oil soluble organic UV blocking agent included in the cosmetic composition, oil soluble organic UV blocking agents modified to be non-adsorbed to the water insoluble sponge are available.

The term "modification" as used herein includes all chemical or physical modifications of the organic UV blocking agent, but may include, for example, methods which prevent a direct contact between the oil soluble organic UV blocking agent present in the external phase and the water insoluble sponge (e.g. encapsulation of the oil soluble organic UV blocking agent), or methods which bring the oil soluble organic UV blocking agent and the water insoluble sponge into contact with each other while not causing an adsorption reaction (e.g. modification of compound structure and modification to water soluble·water dispersible).

According to the present disclosure of the cosmetic of the invention, the cosmetic composition includes:

at least one (or one or more) of the following organic UV blocking agents:

(a) a water soluble or water dispersible organic UV blocking agent; and
(c) an encapsulated organic UV blocking agent, and

the oil soluble organic UV blocking agent is present less than 10%, less than 9 wt%, less than 8 wt%, less than 7 wt%, less than 6 wt%, less than 5 wt%, less than 4 wt%, less than 3 wt%, less than 2 wt%, less than 1 wt%, less than 0.5 wt%, less than 0.05 wt%, less than 0.0005 wt%, or most preferably less than 0.00001 wt% per the total weight of the organic UV blocking agent included in the cosmetic composition.

Also, optionally, the cosmetic composition may further include an inorganic UV blocking agent.

The product with the cosmetic composition according to the present disclosure has an excellent UV blocking effect, preferably with a sun protection factor (SPF) of 30 or above and/or a protection factor of UVA (PFA) of 4 or above, the protection grade of UVA (PA) ++ or above. Also, the cosmetic composition is scarcely sensitive to whitening.

The "sun protection factor (SPF)" stands for an indicator of how effectively products that offer protection against UVB block UVB rays, and is a value obtained by dividing a minimum erythema dose obtained by application of a UV protection product by a minimum erythema dose obtained by non-application of a UV protection product. The "minimum erythema dose (MED)" refers to a minimum dose of UV radiation required to induce erythema over the whole radiation area within the range of 16~24 hours after UVB radiation onto the human skin. The sun protection factor measurement method including test subject selection, test sites, minimum erythema dose measurement at unprotected region, minimum erythema dose measurement at protected region, light source selection, standard sample, an applied amount, definition of applied area and radiation region, light amount increment, sun protection factor calculation, and sun protection factor labeling method, conforms to Appendix 3, Chapter 2, Sun protection factor (SPF) measurement method in 「Regulation on examination of functional cosmetics」 of the notification of Korea's Ministry of Food and Drug Safety No. 2013-28, effective from April 5, 2013.

The "protection factor of UVA (PFA)" stands for an indicator of how effectively products that offer protection against UVA block UVA rays, and is a value obtained by dividing a minimal persistent pigment darkening dose obtained by applying a UV protection product by a minimal persistent pigment darkening dose obtained by non-application of a UV protection product. The "minimal persistent pigment darkening dose (MPPD)" refers to a minimum dose of UV radiation required to perceive slight darkening over the whole radiation area within the range of 2~4 hours after UVA radiation onto the human skin. Also, the "protection grade of UVA (PF)" stands for the degree of UVA protection. PFA 2 or above is marked with PA, and PFA between 2 and 4 (not including 4) is also referred to as PA+, PFA between 4 and 8 (not including 8) is also referred to as PA++, and PFA 8 or above is also referred to as PA+++ (However, the premise is that the figures to the right of the decimal point is discarded from the PFA value to make it an integer). The protection factor of UVA measurement method, including test subject selection, test subject selection, test sites, minimal persistent pigment darkening dose measurement before testing, minimal persistent pigment darkening dose measurement at protected and unprotected regions, light source selection, standard sample, an applied amount, definition of applied area and radiation region, light amount increment, and protection factor of UVA calculation, conforms to Appendix 3, Chapter 4, Protection factor of UVA measurement method of the notification of 「Regulation on examination of functional cosmetics」 No. 2013-28, effective from April 5, 2013.

The product with the cosmetic composition according to the present disclosure has an excellent UV blocking effect and is scarcely sensitive to whitening, and for example, the inorganic UV blocking agent included in the cosmetic composition according to the present disclosure is present in 15 wt% or less, 14 wt% or less, 13 wt% or less, 12 wt% or less, 11 wt% or less, 10 wt% or less, 9 wt% or less, 8wt% or less, 7 wt% or less, 6 wt% or less, 5 wt% or less, 4 wt% or less, 3 wt% or less, 2 wt% or less, or 1 wt% or less per the total weight of the cosmetic composition. If the inorganic UV blocking agent is present more than 15 wt% per the total weight of the cosmetic composition, a whitening phenomenon will be problematic.

The product with the cosmetic composition according to the present disclosure may further include mixtures of pigments, antioxidants, activators, moisturizers, pharmaceuticals, metal ion sequestrants, polyalcohols, preservatives, and flavoring that can be easily made by those skilled in the art without departing from the inhibitory effect for adsorption to the water insoluble sponge, if necessary.

The preparation of the cosmetic composition according to the present disclosure may conform to a general method known in the art in consideration of the type, amount and formulation of the components.

Also, the impregnation of the cosmetic composition according to the present disclosure into the water insoluble sponge may conform to a general method known in the art in consideration of the type and amount of the components included in the cosmetic composition, the formulation of the cosmetic composition, or the type of the water insoluble sponge.

The cosmetic composition (or cosmetics) according to the present disclosure includes, but is not limited to, for example, makeup primer, makeup base, foundation, skin cover, lipstick, lip gloss, face powder, lip liner pencil, eye brow pencil, eye shadow, cheek color, compact powder, twin cake, pact, powder pact, eye brow, eye shadow, concealer, blusher, powder foundation or airless.

[Water insoluble sponge]

The water insoluble sponge used in the cosmetic product according to the present disclosure is formed of a hydrophobic monomer polymer.
Those generally used in the art for cosmetic composition impregnation, include for example, those obtained by foaming a polymer such as styrene-butadiene rubber (SBR), butadiene rubber (BR), natural rubber (NR), acrylonitrile-butadiene rubber (NBR), acrylonitrile-butadiene styrene (ABS), polyethylene (PE), polyvinyl alcohol (PVA), ethylene vinyl acetate (EVA), and polyurethane (PU).

The water insoluble sponge obtained by foaming a hydrophobic monomer polymer has a noticeably superior inhibitory effect for adsorption of the cosmetic composition to the water insoluble sponge according to the present disclosure, and the hydrophobic monomer polymer includes, for example, styrene-butadiene rubber (SBR), butadiene rubber (BR), acrylonitrile-butadiene rubber (NBR), and polyurethane (PU).

A process of impregnating the cosmetic composition according to the present disclosure into the water insoluble sponge may conform to a general method known in the art.

[Cosmetic Product]

The present disclosure provides a cosmetic product including a water insoluble sponge and a cosmetic composition to be impregnated in the water insoluble sponge.

The description of the water insoluble sponge and the cosmetic composition impregnated therein is replaced with the above description to avoid an overlap.

Particularly, the cosmetic composition (of the product according to the invention) to be impregnated in said water insoluble sponge includes (comprises):

at least one (or one or more) of the following organic UV blocking agents:

(a) a water soluble or water dispersible organic UV blocking agent; and
(c) an encapsulated organic UV blocking agent, and

the oil soluble organic UV blocking agent is present less than 10%, less than 9 wt%, less than 8 wt%, less than 7 wt%, less than 6 wt%, less than 5 wt%, less than 4 wt%, less than 3 wt%, less than 2 wt%, less than 1 wt%, less than 0.5 wt%, less than 0.05 wt%, less than 0.0005 wt%, or most preferably less than 0.00001 wt% per the total weight of the organic UV blocking agent included in the cosmetic composition.

The formulation of said cosmetic composition (of the product according to the invention) is a water-in-oil emulsion and said water insoluble sponge is formed of a hydrophobic monomer polymer cosmetic composition may further include an inorganic UV blocking agent.

Also, optionally, the cosmetic composition may further include an inorganic UV blocking agent.

In addition to the water insoluble sponge and the cosmetic composition impregnated therein, the cosmetics according to the present disclosure may include an additional element (e.g. a puff) that can be suitably added by those skilled in the art.

Advantageous Effects

Because the cosmetic composition (in the product) having an ultraviolet (UV) blocking function according to the present disclosure prevents the adsorption of the UV blocking agent to the water insoluble sponge even when impregnated in the water insoluble sponge, there are provided cosmetic products that fully exert an expected UV blocking effect (sun protection factor/grade) from the UV blocking agent included in the cosmetic composition.

BRIEF DESCRIPTION OF THE DRAWINGS

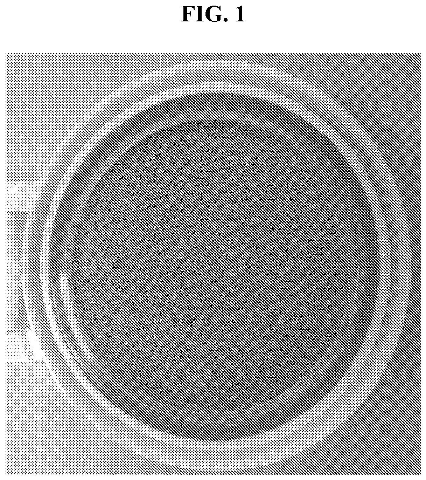

FIG. 1 illustrates cosmetics including foundation impregnated in a water insoluble sponge.

MODE FOR CARRYING OUT THE INVENTION

Hereinafter, the present disclosure will be described in detail with reference to examples and embodiments to aid in understanding the present disclosure. The examples and embodiments of the present disclosure are provided to help one having ordinary skill in the art understand the present disclosure more fully.

<Example> Preparation of water-in-oil type foundation including an organic ultraviolet (UV) blocking agent

Water-in-oil type foundations were prepared with the components and amounts described in the following table 1.

In comparative example 1, water-in-oil type foundation was prepared, including an oil soluble organic UV blocking agent (ethylhexylmethoxycinnamate (EHMC), isoamyl-P-methoxycinnamate (IAMC), ethylhexylsalicylate (EHS)) and an inorganic UV blocking agent (titanium dioxide), while in example 1 and example 2, water-in-oil type foundations were prepared, including a water soluble organic UV blocking agent (phenylbenzimidazolsulfonic acid (PBSA)) and an inorganic UV blocking agent (titanium dioxide).

The water-in-oil type foundations were prepared as below:

Oil phase components and a thickening agent were put in an oil phase tank and heated to 80°C to turn into a uniform state, and pigments were added and dispersed. Water phase components were put in a water phase tank and heated to 80°C to completely dissolve the raw materials, and then were added to the oil phase tank containing the dispersed pigments, followed by emulsification using a homo mixer to prepare a low viscosity UV blocking emulsion. A 40 ml stabilization container was filled with the contents and kept at a 25°C chamber for one day or longer, and after operation of Brookfield LVII viscometer at 30 rpm for 1 minute using spindle 4, when viscosity was measured at 25°C, the viscosity was found to be 4,000 cps.

[Table 1]

| Classification | Name of raw material | Amount (g) | | | |
|---|---|---|---|---|---|
| | | Composition example 1 | Composition example 2 | Example 1 | Example 2 |
| Oil phase component | Cyclopentasiloxane | 17.0 | 20.0 | 25.0 | 25.0 |
| | Phenyl trimethicone | 10.0 | 10.0 | 10.0 | 10.0 |
| | Caprylic/capric triglyceride | 2.0 | 2.0 | 2.0 | 2.0 |
| | Dimethicone | 2.0 | 2.0 | 2.0 | 2.0 |
| | Ethylhexylmethoxycinnamate | 7.5 | 7.5 | 0.0 | 0.0 |
| | PEG-10 dimethicone | 3.0 | 3.0 | 3.0 | 3.0 |
| | Isoamyl-P-methoxycinnamate | 0.5 | 0.5 | 0.0 | 0.0 |
| | Ethylhexylsalicylate | 3.0 | 3.0 | 0.0 | 0.0 |
| | Sorbitan sesquioleate | 1.0 | 1.0 | 1.0 | 1.0 |
| Thickening agent | Disteardimonium hectorite | Optimum amount | Optimum amount | Optimum amount | Optimum amount |
| Pigment | Titanium dioxide (for UV blocking) | 8.0 | 4.0 | 4.0 | 8.0 |
| | Titanium dioxide (pigment grade) | 7.0 | 7.0 | 11.0 | 7.0 |
| | Mica | 2.0 | 2.0 | 2.0 | 2.0 |
| | Yellow iron oxide | 0.9 | 0.9 | 0.9 | 0.9 |
| | Red iron oxide | 0.2 | 0.2 | 0.2 | 0.2 |
| | Black iron oxide | 0.1 | 0.1 | 0.1 | 0.1 |

(continued)

| Classification | Name of raw material | Amount (g) | | | |
|---|---|---|---|---|---|
| | | Composition example 1 | Composition example 2 | Example 1 | Example 2 |
| Water phase | Water | to 100 | to 100 | to 100 | to 100 |
| | Dipropylene glycol | 5.0 | 5.0 | 5.0 | 5.0 |
| | Salt | 1.0 | 1.0 | 1.0 | 1.0 |
| | Tromethamine | 0.0 | 0.0 | 2.5 | 2.5 |
| | Phenylbenzimidazole sulfonic acid | 0.0 | 0.0 | 4.0 | 4.0 |

Experimental example> Adsorption test of a UV blocking agent to a water insoluble sponge

15 g of each foundation of comparative examples 1-2 and examples 1-2 was impregnated in SBR, NBR, and (comparative) PU, and kept at a 25° chamber for one day or longer. After removal of foundation not impregnated in the sponge surface, the following process was performed.

First, each sponge in which the foundations of comparative examples 1-2 and examples 1-2 were impregnated was squeezed by hands to collect the foundation.

According to the functional cosmetics standard and testing method (KFCC) of the notification of Ministry of Food and Drug Safety No. 2013-28, each of the foundation before impregnation into the sponge and the foundation collected from the sponge was dissolved in solvent and tested using an ultraviolet absorption spectrometer according to liquid chromatography to determine a peak area AT of ethylhexylmethoxycinnamate (EHMC), phenylbenzimidazolsulfonic acid (PBSA), ethylhexylsalicylate (EHS), and isoamyl P-methoxycinnamate (IAMC) and a peak area As of a reference standard, and a changed value before and after impregnation was analyzed.

$$\text{an amount of ethylhexylmethoxycinnamate (mg)} = AT/As \times \text{an amount of ethylhexylmethoxycinnamate reference standard (mg)}$$

$$\text{an amount of phenylbenzimidazolsulfonic acid (mg)} = AT/As \times \text{an amount of phenylbenzimidazolsulfonic acid reference standard (mg)}$$

$$\text{an amount of ethylhexylsalicylate (mg)} = AT/As \times \text{an amount of ethylhexylsalicylate reference standard (mg)}$$

$$\text{an amount of isoamyl-P-methoxycinnamate (mg)} = AT/As \times \text{an amount of isoamyl-P-methoxycinnamate reference standard (mg)}$$

The results are as shown in Table 2 (comparative example 1) and Table 3 (example 1).

[Table 2]

| Sponge | EHMC | | | IAMC | | | EHS | | | PBSA | | |
|---|---|---|---|---|---|---|---|---|---|---|---|---|
| | Amount applied (g) | Impregnation (%) | | Amount applied (g) | Impregnation (%) | | Amount applied (g) | Impregnation (%) | | Amount applied (g) | Impregnation (%) | |
| | | before | after | | before | After | | before | after | | before | after |
| SBR | 7.5 | 100.7 | 60.5 | 0.5 | 102.6 | 78.3 | 3.0 | 105.7 | 71.5 | - | - | - |
| NBR | | | 70.4 | | | 82.1 | | | 77.2 | | | - |
| PU | | | 95.1 | | | 93.3 | | | 94.6 | | | - |

EP 3 072 500 B1

[Table 3]

| Sponge | EHMC | | | IAMC | | | EHS | | | PBSA | | |
|---|---|---|---|---|---|---|---|---|---|---|---|---|
| | Amount applied (g) | Impregnation (%) | | Amount applied (g) | Impregnation (%) | | Amount applied (g) | Impregnation (%) | | Amount applied (g) | Impregnation (%) | |
| | | before | after | | before | After | | before | after | | before | after |
| SBR | - | - | - | - | - | - | - | - | - | 4 | 101.3 | 98.8 |
| NBR | | | - | | | - | | | - | | | 102.5 |
| PU | | | - | | | - | | | - | | | 100.6 |

Also, each of the foundations of comparative examples 1-2 and examples 1-2 was uniformly applied to artificial skin (vitroskin) using the impregnated sponges, and after being left behind for 10 minutes, SPF and PFA values were measured using a sun protection factor measuring instrument (SPF-290S).

The results are as shown in Table 4 (in-vitro SPF and in-vitro PFA values of comparative examples 1-2 and examples 1-2).

[Table 4]

| | Comparative example 1 | | Comparative example 2 | | Example 1 | | Example 2 | |
|---|---|---|---|---|---|---|---|---|
| | SPF | PFA | SPF | PFA | SPF | PFA | SPF | PFA |
| Before impregnation | 53.0 | 9.2 | 34.4 | 4.3 | 33.4 | 4.6 | 57.8 | 9.5 |
| SBR | 37.3 | 6.4 | 18.6 | 3.2 | 32.9 | 4.3 | 56.4 | 9.1 |
| NBR | 42.8 | 7.2 | 21.5 | 3.3 | 34.1 | 4.6 | 56.7 | 9.4 |
| PU | 50.3 | 8.6 | 28.3 | 3.7 | 33.2 | 4.5 | 57.3 | 9.2 |

As a result of the adsorption test of the UV blocking agent to the water insoluble sponge, it could be seen that when the oil soluble organic UV blocking agent such as ethylhexylmethoxycinnamate, isoamyl-P-methoxycinnamate and ethylhexylsalicylate is present in oil phase (comparative examples 1-2), the collected amount greatly reduced due to the adsorption to the water insoluble sponge, resulting in a significant reduction in sun protection factor, and it could be seen that the main component amount is less than 90% that is below the standard under the UV blocking functional cosmetics Act, causing a legal problem to occur. In contrast, it was found that when the water soluble or water dispersible UV blocking agent such as phenylbenzimidazolsulfonic acid is used (examples 1-2), it was not adsorbed to the water insoluble sponge and was detected well. In addition, it could be seen that an excellent UV blocking effect with SPF of 30 or above and PFA of 4 or above was produced.

In the case of the water insoluble sponge, adsorption of the UV blocking agent greatly took place in SBR or NBR, and in the case of (comparative) PU, adsorption of the UV blocking agent took place but the degree of adsorption was relatively low as compared to SBR or NBR, and when impregnation into the SBR or NBR sponge, a higher UV blocking effect could be obtained.

Industrial Applicability

Because the cosmetic composition having an ultraviolet (UV) blocking function according to the present disclosure prevents the adsorption of the UV blocking agent to the water insoluble sponge even when impregnated in the water insoluble sponge, there are provided cosmetics that fully exert an expected UV blocking effect (sun protection factor/grade) from the UV blocking agent included in the cosmetic composition.

**Claims**

1. A cosmetic product comprising a water insoluble sponge and a cosmetic composition to be impregnated in said water insoluble sponge,
   wherein said cosmetic composition comprises one or more of the following UV blocking agents:

   (i) a water soluble or water dispersible organic UV blocking agent; and
   (ii) an encapsulated organic UV blocking agent,

   and an oil soluble organic UV blocking agent is present at less than 10 wt% per total weight of the organic ultraviolet light blocking agent included in the cosmetic composition,
   wherein the formulation of the cosmetic composition is a water-in-oil emulsion,
   wherein said water insoluble sponge is formed of a hydrophobic monomer polymer.

2. The cosmetic product according to claim 1, wherein the cosmetic composition has a sun protection factor (SPF) of 30 or above and protection grade of UVA (PA) ++ or above.

**3.** The cosmetic product according to claim 1, wherein the cosmetic composition further comprises an inorganic UV blocking agent.

**4.** The cosmetic product according to claim 1, wherein the hydrophobic monomer polymer is styrene-butadiene rubber (SBR), butadiene rubber (BR), acrylonitrile rubber (NR) or acrylonitrile-butadiene rubber (NBR).

**5.** The cosmetic product according to claim 1, wherein the cosmetic composition is foundation.

**Patentansprüche**

**1.** Kosmetisches Produkt umfassend einen wasserunlöslichen Schwamm und eine kosmetische, im wasserunlöslichen Schwamm zu imprägnierende Zusammensetzung,
wobei die kosmetische Zusammensetzung ein oder mehrere der folgenden UV-Blockierungsmittel umfasst:

(i) ein wasserlösliches oder wasserdispergierbares organisches UV-Blockierungsmittel; und
(ii) ein verkapseltes organisches UV-Blockierungsmittel,

und ein öllösliches organisches UV-Blockierungsmittel, vorliegend als weniger als 10 Gew.-% des Gesamtgewichts des organischen Ultraviolettlichtblockierungsmittels in der kosmetischen Zusammensetzung,
wobei die Formulierung der kosmetischen Zusammensetzung eine Wasser-in-Öl-Emulsion ist,
wobei der wasserunlösliche Schwamm aus einem hydrophoben Monomerpolymer gebildet ist.

**2.** Kosmetisches Produkt nach Anspruch 1, wobei die kosmetische Zusammensetzung einen Lichtschutzfaktor (LSF) von 30 oder mehr und einen UVA (PA) ++-Schutzgrad oder mehr hat.

**3.** Kosmetisches Produkt nach Anspruch 1, wobei die kosmetische Zusammensetzung weiter ein anorganisches UV-Blockierungsmittel umfasst.

**4.** Kosmetisches Produkt nach Anspruch 1, wobei das hydrophobe Monomerpolymer Styrol-Butadien-Kautschuk (SBR), Butadien-Kautschuk (BR), Acrylonitril-Kautschuk (NR) oder Acrylonitril-Butadien-Kautschuk (NBR) ist.

**5.** Kosmetisches Produkt nach Anspruch 1, wobei die kosmetische Zusammensetzung eine Foundation ist.

**Revendications**

**1.** Composition cosmétique comprenant une éponge insoluble dans l'eau et une composition cosmétique à imprégner dans ladite éponge insoluble dans l'eau,
dans laquelle ladite composition cosmétique comprend un ou plusieurs des agents bloquant les UV suivants :

(i) un agent organique bloquant les UV soluble dans l'eau ou dispersable dans l'eau ; et
(ii) un agent organique bloquant les UV encapsulé,

et un agent organique bloquant les UV soluble dans l'huile est présent à moins de 10 % en poids par rapport au poids total de l'agent organique bloquant la lumière ultraviolette compris dans la composition cosmétique,
dans laquelle la formulation de la composition cosmétique est du type huile dans l'eau,
dans laquelle l'éponge insoluble dans l'eau est formée d'un polymère monomère hydrophobe.

**2.** Composition cosmétique selon la revendication 1, dans laquelle la composition cosmétique a un facteur de protection solaire (FPS) de 30 ou plus et un degré de protection d'UVA (PA) ++ ou plus.

**3.** Composition cosmétique selon la revendication 1, dans laquelle la composition cosmétique comprend en outre un agent inorganique bloquant les UV.

**4.** Composition cosmétique selon la revendication 1, dans laquelle le polymère monomère hydrophobe est du caoutchouc styrène-butadiène (SBR), du caoutchouc butadiène (BR), du caoutchouc acrylonitrile (NR) ou du caoutchouc acrylonitrile-butadiène (NBR).

**5.** Composition cosmétique selon la revendication 1, dans lequel la composition cosmétique est un fond de teint.

## FIG. 1

**EP 3 072 500 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20130116205 A
- US 2015118269 A1
- KR 201328
- WO 201328 A